(19) Europäisches Patentamt
European Patent Office
Office européen des brevets

(11) **EP 1 904 099 B1**

(12) **FASCICULE DE BREVET EUROPEEN**

(45) Date de publication et mention
de la délivrance du brevet:
**11.03.2009 Bulletin 2009/11**

(51) Int Cl.:
***A61K 39/39*** *(2006.01)* ***A61K 39/145*** *(2006.01)*
***A61K 9/107*** *(2006.01)*

(21) Numéro de dépôt: **06778810.9**

(22) Date de dépôt: **07.07.2006**

(86) Numéro de dépôt international:
**PCT/FR2006/001635**

(87) Numéro de publication internationale:
**WO 2007/006939 (18.01.2007 Gazette 2007/03)**

(54) **EMULSION IMMUNO-ADJUVANTE THERMORÉVERSIBLE**

THERMOREVERSIBLE IMMUNADJUVANSEMULSION

THERMOREVERSIBLE IMMUNO- ADJUVANT EMULSION

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **07.07.2005 FR 0507240**
**04.08.2005 FR 0508310**

(43) Date de publication de la demande:
**02.04.2008 Bulletin 2008/14**

(73) Titulaire: **Sanofi Pasteur**
**69367 Lyon Cedex 07 (FR)**

(72) Inventeurs:
• **KLUCKER, Marie-Françoise**
**F-69300 Caluire et Cuire (FR)**
• **DALENCON, François**
**F-69004 Lyon (FR)**
• **PROBECK-QUELLEC, Patricia**
**F-69007 Lyon (FR)**

(74) Mandataire: **Kerneis, Daniéle et al**
**Sanofi Pasteur**
**Direction de la Propriété Intellectuelle**
**2, Avenue Pont Pasteur**
**69367 Lyon Cedex 07 (FR)**

(56) Documents cités:
**US-A1- 2003 022 852** **US-A1- 2003 060 559**
**US-B1- 6 299 884** **US-B1- 6 544 518**
**US-B1- 6 787 523**

**Description**

La présente invention est relative au domaine des vaccins; plus particulièrement, l'invention est relative au domaine des vaccins comprenant une émulsion adjuvante. Il existe dans l'art antérieur de nombreux vaccins qui contiennent un ou plusieurs adjuvants. Le brevet US 6 299 884 décrit notamment une formulation adjuvante comprenant une émulsion huile-dans-eau, dont la taille des gouttelettes d'huile est comprise entre 100 et 1000 nm. Cette émulsion est obtenue au moyen d'un homogénéiseur haute pression (microfluidiseur), au cours d'un procédé de fabrication mettant en oeuvre de hautes énergies mécaniques afin d'obtenir des forces de cisaillement suffisamment importantes pour réduire la taille des gouttes d'huile. Selon cet enseignement, si la valeur minimale de la fourchette des tailles des gouttes obtenues est de 100 nm, la valeur moyenne est nettement plus élevée et se situe au mieux vers 170 nm, plus généralement vers 500 nm.

Il est souhaitable de pouvoir disposer d'une formulation alternative à celle proposée dans ce brevet, qui puisse être obtenue par un procédé plus simple (ne nécessitant pas une technologie de cisaillement particulière), et de basse énergie tout en étant reproductible et parfaitement fiable ; en outre, la formulation adjuvante doit permettre d'adjuver efficacement les vaccins, en permettant notamment d'augmenter la réponse immunitaire obtenue ou de diminuer la dose d'antigène présente, tout en ne présentant pas de signe de toxicité qui nuirait à son administration en toute sécurité. Pour atteindre ce but, la présente invention a pour objet une émulsion adjuvante huile-dans-eau caractérisée en ce qu'elle comprend au moins :

- du squalène,
- un solvant aqueux,
- un tensioactif non-ionique hydrophile qui est un polyoxyéthylène alkyl éther,
- un tensioactif non ionique hydrophobe,

en ce qu'elle est thermoréversible, et en ce que 90% de la population volumique des gouttes d'huile a une taille inférieure à 200 nm. Selon l'invention, une telle émulsion peut être obtenue par un procédé d'inversion de phase par variation de température, ce qui procure un très gros avantage d'un point de vue industriel. Un tel procédé présente toutes les garanties de sécurité et de rentabilité nécessaires à l'industrie pharmaceutique. En outre, grâce à ce procédé, il est possible d'obtenir une émulsion monodisperse dont la taille des gouttelettes d'huile est très faible, ce qui rend l'émulsion ainsi obtenue particulièrement stable et facilement filtrable au moyen de filtres stérilisants dont le seuil de coupure est de 200 nm.

Selon une caractéristique particulière, 90% de la population volumique (ou d90) des gouttes d'huile a une taille inférieure à 160 nm, et même à 150 nm.

Selon un mode particulier de réalisation de l'invention, l'émulsion selon l'invention comprend en outre un alditol ; ceci permet d'obtenir une inversion de phase à une température inférieure à celle qui serait nécessaire pour la même composition ne présentant pas d'alditol, ce qui permet de réduire les coûts de production ainsi que les risques de dénaturation thermique des constituants de l'émulsion.

Selon un mode particulièrement avantageux, le tensioactif non-ionique hydrophobe de l'invention est un ester de sorbitanne ou un ester de mannide. De tels tensioactifs présentent l'avantage de pouvoir être utilisés en toute sécurité dans les solutions injectables.

Selon un mode particulier de l'invention, l'émulsion comprend en outre un alkylpolyglycoside et un agent cryoprotecteur tel qu'un sucre, en particulier du dodécylmaltoside et/ou du saccharose.

Ainsi, il est possible d'obtenir une émulsion lyophilisable qui, après lyophilisation et reconstitution, retrouve ses propriétés, notamment granulométriques, c'est-à-dire que l'émulsion lyophilisée puis reconstituée est toujours monodisperse et est constituée de gouttelettes d'huile dont 90% de la population volumique a une taille inférieure à 200 nm. Ceci est particulièrement important pour le domaine des vaccins qui doivent parfois pour des raisons de stabilité (soit de certains antigènes, soit de certains adjuvants) être conservés sous forme lyophilisée.

L'invention a également pour objet un procédé de préparation d'une composition immunogène comprenant au moins un antigène vaccinal et une émulsion huile-dans-eau, caractérisé en ce que l'huile est du squalène et en ce que l'émulsion huile-dans-eau est obtenue par un procédé d'inversion de phase par variation de la température.

Selon un mode de réalisation, le procédé selon l'invention comprend au moins une étape de préparation de l'émulsion huile-dans-eau par refroidissement d'une émulsion inverse eau-dans-huile qui comprend au moins :

- du squalène,
- un solvant aqueux,
- un tensioactif non ionique hydrophile qui est un polyoxyéthylène alkyl éther,
- un tensioactif non ionique hydrophobe.

Grâce à un tel procédé, très avantageux d'un point de vue industriel, on obtient une composition immunogène stable, très efficace même avec une très faible dose d'antigènes.

En outre, grâce au procédé de préparation utilisé, les gouttelettes d'huile de l'émulsion sont toutes calibrées sur une même très petite taille; en effet, lorsqu'on réalise la mesure , des propriétés granulométriques (taille et distribution de taille), on remarque que l'on a une émulsion monodisperse, avec une courbe de distribution de type gaussienne, très resserrée, centrée autour d'une valeur faible, généralement autour de 80-90 mm.

Selon une mise en oeuvre particulière du procédé selon l'invention, on obtient l'émulsion inverse eau-dans-huile en mélangeant du squalène, un solvant aqueux, un tensioactif non ionique hydrophile qui est du polyoxyéthylène alkyl éther, et un tensioactif non ionique hydrophobe afin d'obtenir tout d'abord une émulsion grossière huile-dans-eau, et on chauffe ensuite cette émulsion jusqu'à au moins la température d'inversion de phase afin d'obtenir l'émulsion inverse. Une telle façon de procéder a pour avantage de limiter la durée pendant laquelle les différents composants de l'émulsion seront soumis à une température élevée.

Selon un autre mode de mise en oeuvre, le procédé selon l'invention comprend les étapes suivantes :

- on chauffe, séparément, à une température au moins égale à la température d'inversion de phase, d'une part la phase aqueuse comprenant le solvant aqueux et le polyoxyéthylène alkyl éther, et d'autre part la phase huileuse comprenant le squalène et le tensioactif hydrophobe puis,
- on mélange les 2 phases afin d'obtenir une émulsion inverse eau-dans huile.

Selon un mode particulier de réalisation de l'invention, chacune des phases aqueuse et huileuse est chauffée séparément, avant mélange, à une température inférieure à la température d'inversion de phase. Puis les 2 phases sont mélangées afin d'obtenir une émulsion huile-dans-eau ; l'ensemble est ensuite chauffé à une température au moins égale à la température d'inversion de phase afin d'obtenir l'émulsion inverse eau-dans-huile.

Selon un mode de réalisation particulier, le procédé de préparation selon l'invention comprend en outre une étape de lyophilisation. Ainsi, le procédé selon l'invention peut être utilisé pour la préparation de compositions immunogènes comprenant des antigènes qui doivent être conservés sous forme lyophilisée pour des raisons de stabilité.

De nombreux autres avantages de la présente invention apparaîtront au cours de la description qui va suivre.

Aux fins de description de l'invention, les paramètres de d50 et d90 mentionnés dans la présente demande de brevet, sont des valeurs volumiques ; la valeur de d50 signifie la valeur de 50% de la population volumique des gouttes.

Par émulsion huile-dans-eau au sens de l'invention, on entend une dispersion d'une phase huileuse dans une phase aqueuse qui peut être constituée soit d'eau, soit d'une solution saline, éventuellement tamponnée. Selon un mode particulier de réalisation de l'invention, la phase aqueuse de l'émulsion est constituée par un tampon, tel que les solutions de Dulbecco tamponnées au Phosphate (D-PBS, sans calcium ni magnésium). Par émulsion « adjuvante », on entend une émulsion immuno-adjuvante, c'est-à-dire capable de modifier la réponse du système immunitaire induite lors de l'administration d'un antigène, par rapport à la réponse obtenue en absence de l'émulsion ; cette réponse du système immunitaire peut se traduire par une production d'anticorps ou par une activation de certaines cellules, notamment les cellules présentatrices d'antigènes (par exemple les cellules dendritiques), les lymphocytes T, les lymphocytes B. Cette activation cellulaire peut être mise en évidence par la présence de marqueurs d'activation à la surface des cellules ou par la libération de cytokines. La modification de la réponse immunitaire induite par l'émulsion adjuvante peut être de nature quantitative, c'est-à-dire que l'on obtient une augmentation de la réponse induite, ou de nature qualitative, c'est-à-dire que l'on obtient une réponse de nature ou d'orientation différente, ou encore que l'on obtient une réponse supplémentaire. Par émulsion adjuvante, on entend également une émulsion qui permet de réduire la quantité d'antigènes administrés pour une même réponse induite.

Par composition immunogène au sens de la présente invention, on entend une composition comprenant au moins un antigène et susceptible d'être administrée à l'homme ou à l'animal afin d'induire une réponse du système immunitaire. Cette réponse peut être une réponse humorale (production d'anticorps) ou cellulaire (prolifération et/ou activation de cellules immunitaires). La composition immunogène peut être une composition à visée prophylactique ou à visée thérapeutique, ou encore les deux. La composition immunogène obtenue selon l'invention peut être administrée par toutes les voies habituellement utilisées ou préconisées pour les vaccins : voie parentérale, voie muqueuse, ...et se présenter sous diverses formes notamment liquide ou lyophilisée. Elle peut être administrée au moyen d'une seringue ou au moyen d'un injecteur sans aiguille pour injection intramusculaire, sous-cutanée ou intradermique, ou au moyen d'un spray nasal.

Par antigène au sens de la présente invention, on entend tout antigène susceptible d'être utilisé dans un vaccin, qu'il s'agisse d'un germe entier ou d'un antigène sous-unitaire, peu important sa nature ; l'antigène peut en effet être un peptide, une protéine, une glycoprotéine, un polysaccharide, un glycolipide, un lipopeptide...etc L'émulsion adjuvante selon l'invention est particulièrement adaptée aux antigènes viraux ; on a en effet obtenu de particulièrement bons résultats avec des antigènes du cytomégalovirus humain, du virus de l'immunodéficience humaine, et de la grippe. En ce qui concerne les antigènes du virus de la grippe, il est possible d'utiliser des antigènes provenant d'une seule souche virale, ou d'un mélange de différentes souches. Il est possible d'utiliser des antigènes issus de virus cultivés de façon traditionnelle sur oeufs, ou sur cellules. Grâce à l'invention, on a remarqué qu'on pouvait, que ce soit pour une seule souche ou pour un mélange de souches, obtenir une réponse satisfaisante du système immunitaire tout en réduisant très fortement la quantité d'antigènes présente dans la dose vaccinale. Ceci peut être d'un intérêt particulièrement grand dans le cas de la préparation d'un vaccin contre une pandémie de grippe, où il faut pouvoir produire dans un délai très court, des quantités très importantes de doses vaccinales.

Selon l'invention, l'émulsion huile-dans-eau comprend du squalène qui est une huile provenant à l'origine du foie de requin ; c'est une huile dont la formule chimique brute est $C_{30}H_{50}$, comprenant 6 double-liaisons, cette huile est métabolisable et présente des qualités permettant son utilisation dans un produit pharmaceutique injectable. Il existe également du squalène d'origine végétale extrait de l'huile d'olive. On a notamment obtenu de bons résultats en utilisant le squalène fourni par la société Fluka qui est d'origine animale. Les quantités de squalène utilisées pour la préparation d'une émulsion concentrée sont avantageusement comprises entre 5 et 45 % ; cette émulsion concentrée est ensuite diluée lors de la préparation des compositions immunogènes pour préparer des doses immunisantes dans lesquelles la quantité de squalène sera comprise entre 0,5 et 5%. Cette dilution peut être effectuée par simple mélange de l'émulsion adjuvante selon l'invention et de la suspension comprenant l'antigène.

Selon l'invention, l'émulsion comprend un tensioactif hydrophile non ionique dont la valeur de la balance hydrophile/lipophile ou HLB est supérieure ou égale à 10 et qui appartient au groupe chimique des polyoxyéthylène alkyl éthers (PAE), encore appelés éthers polyoxyéthylénés d'alcools gras, ou polyoxyéthylène glycol éthers de n-alcool. Ces tensioactifs non ioniques sont obtenus par condensation chimique entre un alcool gras et l'oxyde d'éthylène. Ils ont une formule chimique générale du type $CH_3$ $(CH_2)_x$-$(O-CH_2-CH_2)_n$ -OH dans laquelle n désigne le nombre d'unités d'oxyde d'éthylène et est habituellement compris entre 10 et 60 et x +1 est le nombre de carbone, fonction des alcools gras utilisés. En général, ces produits sont des mélanges de polymères avec des chaînes hydrocarbonées de longueur proche. L'émulsion selon l'invention comprend habituellement un seul PAE hydrophile. Un mélange de plusieurs PAE convient également dans la mesure où la valeur globale de HLB est ≥ 10.

Les éthers polyoxyéthylénés d'alcools gras convenant à l'objet de l'invention peuvent être sous une forme liquide ou solide à température ambiante. Parmi les composés solides, on préféré ceux qui se dissolvent directement dans la phase aqueuse ou qui ne nécessitent pas de chauffage important.

Dans la mesure où le nombre d'unités d'oxyde d'éthylène est suffisant, les éthers polyoxyéthylénés d'alcools laurique, myristique, cétylique, oléique et/ou stéarique sont particulièrement appropriés à l'objet de l'invention. On peut notamment les trouver dans la gamme des produits connus sous les appellations commerciales de Brij ® pour les produits commercialisés par sa Société ICI America's Inc., Eumulgin ® pour les produits commercialisés par la Société Cognis, ou Simulsol ® pour les produits commercialisés par la Société SEPPIC.

Une émulsion particulièrement préférée selon l'invention contient comme tensioactif hygrophile non ionique un polyoxyéthylène alkyl éther choisi dans le groupe constitué du ceteareth-12 (commercialisé sous la dénomination d'Eumulgin ® B1), du ceteareth-20 (Eumulgin ® B2), du steareth-21 (Eumulgin ® S21), du ceteth-20 (Simulsol ® 58 ou Brij ®58), du ceteth-10 (Brij ®56), du steareth-10 (Brij ®76), du steareth-20 (Brij ®78), du oleth-10 (Brij ®96 ou Brij ®97), du oleth-20 (Brij ®98 ou Brij ®99). Le nombre apposé à chaque nom chimique correspond au nombre d'unités d'oxyde d'éthylène dans la formule chimique.

On a obtenu de bons résultats avec le produit BRIJ® 56. Un composé particulièrement adapté et préféré en raison de son origine semi-synthétique est le polyoxyéthylène (12) éther cétostéarylique, fourni par la Société COGNIS sous la dénomination d'Eumulgin™B1. Ce composé est un mélange de $CH_3$ $(CH_2)_{15}$-$(O-CH_2-CH_2)_{12}$ -OH et de $CH_3$ $(CH_2)_{17}$-$(O-CH_2-CH_2)_{12}$-OH.

Selon l'invention, l'émulsion adjuvante comprend également un tensioactif non-ionique hydrophobe ; il doit s'agir d'un tensioactif pouvant être utilisé dans l'industrie pharmaceutique ; parmi les tensioactifs convenant à cet égard, on peut citer les esters sorbitanne ainsi que les esters de mannide ; les esters de sorbitanne sont obtenus par réaction d'un acide gras et de mélange d'esters partiels de sorbitol et ses mono et di-anhydres ; il peut s'agir d'un mono, d'un di ou d'un tri-ester, ou même d'un mélange ; ce sont des tensioactifs hydrophobes dont la balance hydrophilie-lipophilie globale (HLB) est inférieure à 9, et de préférence inférieure à 6. On peut notamment les trouver dans la gamme des tensioactifs commercialisés par la Société ICI America's Inc. sous la dénomination de SPAN®, ou par la Société COGNIS sous la dénomination de Dehymuls™, ou par la Société ICI sous la dénomination de ARLACEL™ ; comme exemples de tensioactifs convenant particulièrement bien, on peut citer le monooléate de sorbitanne, commercialisé sous la dénomination de Dehymuls SMO™ ou de SPAN®80. Parmi les tensioactifs constitués par des esters de mannide, on peut citer le mannide monooléate commercialisé par la Société SIGMA, ou par la Société SEPPIC sous la dénomination de Montanide 80™.

Grâce à la sélection de ces tensioactifs particuliers parmi tous les tensioactifs proposés dans l'art antérieur pour préparer des émulsions, il a maintenant été trouvé que l'on pouvait, très avantageusement, produire une émulsion adjuvante huile-dans-eau en utilisant un procédé d'inversion de phase.

Pour cela, les quantités de squalène et de chacun des tensioactifs utilisés sont avantageusement choisies de manière à obtenir un mélange dont le diagramme de phases comporte une phase de courbure moyenne nulle (type microémulsion ou phase lamellaire) pour laquelle les tensions interfaciales sont extrêmement basses.

Dans le cas de l'utilisation du squalène, on a remarqué que les émulsions obtenues sont stables, monodisperses et avec une taille très petite des gouttelettes d'huile (d90 inférieure à 200 nm), lorsque la valeur globale de HLB des différents tensioactifs utilisés se situe entre 8,5 et 10, et plus particulièrement entre 8,6 et 9,6. Pour déterminer les concentrations respectives en tensioactifs hydrophile et hydrophobe dans la composition de l'émulsion on peut utiliser la formule suivante :

$$HLB_m = (HLB_e \times M) + HLB_{pae}(1-M)$$

dans laquelle,

$HLB_m$ correspond à la HLB du mélange qui est de préférence entre 8,5 et 10, et plus particulièrement entre 8,6 et 9,6

$HLB_e$ correspond à la HLB du tensioactif hydrophobe

M correspond au pourcentage en masse du tensioactif hydrophobe dans le mélange constitué par le tensioactif hydrophobe et le polyoxyéthylène alkyl éther (PAE)

$HLB_{pae}$ correspond à la HLB du PAE

On a remarqué qu'en utilisant une concentration en squalène comprise entre 5 et 45%, on obtenait, de façon très avantageuse, une émulsion dont la température d'inversion de phase était inférieure à 95°C.

Pour une telle émulsion, il est possible d'utiliser un polyoxyéthylène alkyl éther à une concentration comprise entre 0,9 et 9 % et un tensioactif non-ionique hydrophobe à une concentration comprise entre 0,7 et 7% ; le reste de l'émulsion étant constitué par un solvant aqueux.

Selon un mode particulier de réalisation de l'invention, la composition immunogène comprend, en outre, un alditol tel que notamment le glycérol, l'érythritol, le xylitol, le sorbitol ou le mannitol. On a notamment obtenu de bons résultats avec le mannitol commercialisé par la Société ROQUETTE Frères. Les quantités d'alditol utilisées lors du processus de préparation, peuvent varier entre 1 et 10 %, et plus particulièrement entre 2 et 7.

Selon un mode particulier de réalisation de l'invention, l'émulsion adjuvante comprend en outre un cryoprotecteur qui permet la lyophilisation de l'émulsion obtenue ; parmi les cryoprotecteurs, les sucres sont particulièrement préférés, et notamment le saccharose. En outre, l'émulsion selon l'invention peut comprendre un alkylpolyglycoside qui est un tensioactif à tête sucre ; il peut notamment s'agir de décyl-D galactoside uronate de sodium, ou selon un mode préféré de dodécyl-β-maltoside disponible auprès de la Société ROCHE.

Grâce au procédé de préparation de l'émulsion selon l'invention par une inversion de phase obtenue en faisant varier la température, on obtient très facilement, de façon très reproductible, une émulsion huile-dans-eau dont la taille des gouttelettes d'huile est très homogène ; la valeur de d90 (en volume) est inférieure à 200 nm, de préférence inférieure à 150 nm, et même proche de 100 nm, alors que la valeur de d50 est inférieure à 100 nm, ou même 90 nm. La plupart des émulsions préparées selon le procédé de l'invention ont permis d'atteindre des valeurs de d50 autour de 80 nm avec des valeurs de d90 autour de 100 nm (mesures réalisées au Coulter LS230). On peut ainsi, à condition qu'elle soit suffisamment diluée, effectuer une filtration stérilisante de l'émulsion obtenue.

De telles émulsions dont la taille des gouttes est homogène et très petite, sont stables dans le temps. Ainsi, on a pu remarquer qu'une émulsion préparée selon l'invention et stockée à 4°C , conservait après 2 ans, un profil monodisperse , avec une valeur de d50 de 90 nm et une valeur de d90 de 116 nm, ce qui prouve la très grande stabilité de l'émulsion.

La mesure de la taille des gouttes peut se faire par différents moyens, et notamment par des granulomètres à diffraction LASER, tels que les appareils Beckman Coulter de la gamme LS (notamment le LS230), ou des appareils Malvern de la gamme Mastersizer (notamment le Mastersizer 2000). Le principe de mesure de ces appareils est basé sur l'analyse de l'intensité de la lumière diffusée par les particules en fonction de l'angle (détecteurs de grands, moyens et petits angles) lorsque l'échantillon est éclairé par un faisceau LASER. Cette analyse se fait au moyen de modèles mathématiques choisis selon la taille et la nature du matériau utilisé. Dans le cas de mesure de taille de particules submicroniques, il faut appliquer un modèle optique particulier (théorie de Mie) prenant en compte les indices de réfraction de l'échantillon (ici 1,495 pour le squalène) et de son milieu (ici 1,332 pour l'eau) ; il faut également être capable de détecter les faibles intensités émises par les particules très fines, ce qui nécessite une optimisation de l'analyse :

- une cellule supplémentaire de détection pour la mesure aux grands angles de la diffusion différentielle des intensités polarisées (système PIDS chez Coulter qui permet une mesure dès 40 nm,)

- système de détection combinant 2 longueurs d'onde, lumière bleue et rouge, chez Malvern. La source de lumière bleue de plus basse longueur d'onde, associée à des détecteurs de diffusion aux larges angles et en rétrodiffusion renforce les performances de l'analyse dans la gamme submicronique.

Selon les appareils utilisés, les mesures peuvent varier légèrement en fonction des composants de l'appareil et des logiciels de traitement de données utilisées. Ainsi, une même émulsion selon l'invention a été analysée avec les 2 appareils et a donné les résultats suivants :

- au LS230, avec les paramètres suivants : IR particule=1.495 ; IR milieu=1.332 ; valeur d'absorption=0 ; d50=80-90 nm et d90=120-130 nm ;
- au Mastersizer 2000, avec les paramètres suivants : IR particule=1.495 ; IR milieu=1.332 ; valeur d'absorption=0 ; obscuration=4-.7% ; modèle optique « Général purpose » ; d50 = 90-100 nm et d90 =140-150 nm

Le procédé selon l'invention peut être mis en oeuvre de la manière suivante : on prépare une émulsion grossière huile-dans-eau concentrée par incorporation de la phase aqueuse (solution tamponnée, éventuellement additionnée d'alditol, comprenant le polyoxyéthylène alkyl éther) dans la phase huileuse (squalène, et tensioactif non ionique hydrophobe). Ou à l'inverse, par incorporation de la phase huileuse dans la phase aqueuse. On obtient alors une émulsion huile-dans-eau non calibrée, qui manifeste rapidement son instabilité. Cette émulsion est agitée et chauffée jusqu'à obtention d'une inversion de phase, c'est-à-dire l'obtention d'une émulsion eau-dans-huile. La transition ou inversion de phase peut être suivie par conductimétrie. En effet, lors de la montée en température, la conductivité augmente jusqu'à l'inversion de phase ; à ce moment-là, on observe une chute relativement brutale de la conductivité. La température à laquelle se produit le changement de courbure de la courbe de suivi de la conductivité traduit le passage d'un type d'émulsion à un autre ; c'est la température d'inversion de phase. En réalité, cette température est plutôt un intervalle de température qu'une valeur ponctuelle très précise ; en effet, on peut considérer que cette température est une température déterminée à 1 ou 2 degrés près afin que l'ensemble de l'émulsion subisse le phénomène d'inversion de phase. Une fois cette température d'inversion de phase atteinte, et donc en présence d'une émulsion eau-dans-huile, on arrête le chauffage et on refroidit le mélange. Le refroidissement peut être effectué de façon passive, en laissant simplement l'émulsion revenir spontanément à la température ambiante, ou de manière plus active, en effectuant par exemple une trempe de l'émulsion dans un bain de glace. Lors du passage par la température d'inversion de phase, l'émulsion eau-dans-huile va à nouveau s'inverser pour redonner une émulsion huile-dans-eau, dont la taille des gouttelettes d'huile est cette fois très homogène et petite ; l'émulsion obtenue est alors très stable. Elle peut être stockée en l'état en attendant d'être diluée par une solution comprenant l'antigène vaccinal.
Cette émulsion est thermoréversible, ce qui signifie que si elle est portée à nouveau à une température supérieure à la température d'inversion de phase, elle va redevenir une émulsion eau dans l'huile. On remarque que les courbes de suivi de la conductivité sont superposables pour une même émulsion, quel que soit le nombre de thermoinversions subies, et que les émulsions obtenues ont toujours le même profil granulométrique. De façon avantageuse selon l'invention, la formulation de l'émulsion est choisie pour avoir une température d'inversion de phase qui soit inférieure à 95°C, et plus particulièrement comprise entre 45 et 80 °C, et plus particulièrement encore entre 50 et 65°C. Cette gamme de température est avantageuse car l'émulsion ne risque pas de changer d'état si elle est stockée à une température relativement élevée (≈37°C). De plus, comme dans le procédé de préparation de l'émulsion thermoréversible, le chauffage des composants n'est pas trop important, cela contribue au maintien de l'intégrité structurale des composants. Lorsque la température d'inversion de phase de l'émulsion est élevée, notamment lorsqu'elle est supérieure ou voisine de 80°C, on peut utilement l'abaisser en ajoutant à la composition de l'émulsion un alditol qui est habituellement choisi parmi le sorbitol, le mannitol, le glycérol, le xylitol ou l'érythritol. Lorsque l'alditol est utilisé dans une gamme de concentration allant de 1 à 10% (p/p) et en particulier dans une gamme de concentration allant de 2 à 7% (p/p) on arrive à abaisser la température d'inversion de phase de l'émulsion d'environ 10°C. On peut également abaisser la température d'inversion de phase de l'émulsion, en remplaçant la phase aqueuse constituée uniquement d'eau par une phase aqueuse saline tamponnée. Habituellement, on utilise un tampon TRIS, ou un tampon phosphate comme le PBS ou le tampon Dulbecco PBS sans $Ca^{2+}$ ni $Mg^{2+}$.

Il existe des alternatives au procédé qui vient d'être décrit. En effet, il est possible, ainsi que cela vient d'être décrit, de mélanger les 2 phases aqueuse et huileuse afin d'obtenir l'émulsion brute qui sera alors chauffée puis refroidie. Alternativement, les 2 phases préparées peuvent être chauffées séparément à une température légèrement supérieure à la température d'inversion de phase, avant d'être mélangées pour donner une émulsion inverse eau-dans-huile qui sera refroidie jusqu'à obtention de l'émulsion submicronique huile-dans-eau. On peut également chauffer légèrement chacune des phases avant de faire le mélange qui conduira à une émulsion huile-dans-eau, puis chauffer cette émulsion jusqu'à l'inversion de phase avant de procéder au refroidissement.

Toutes ces opérations peuvent être réalisées dans des réservoirs séparés pour une préparation en lots, mais il est également possible d'utiliser un procédé en ligne.

Le procédé de préparation de l'émulsion en ligne peut notamment consister en un mélange à chaud des deux phases aqueuse et huileuse préalablement préparées séparément, au travers d'un mélangeur statique thermostaté suivi d'un refroidissement en ligne au travers d'un échangeur thermique réfrigéré connecté en sortie du mélangeur statique, puis de la récupération finale de l'émulsion selon l'invention dans un récipient approprié (flacon ou réacteur).

On a utilisé avec succès un mélangeur statique constitué d'une succession d'éléments de mélange composés de lames croisées et inclinées par rapport à l'axe du tube dans lequel ils sont introduits. L'énergie nécessaire au mélange est fournie par les pompes qui véhiculent les fluides et le mélange est réalisé sans pièce mobile, au travers des éléments de mélange par la séparation, le déplacement et la réunion successive des constituants du mélange.

Le procédé de préparation en ligne est mis en oeuvre de la manière suivante : on prépare séparément la phase aqueuse (solution tamponnée comprenant le polyoxyéthylène alkyl éther) et la phase huileuse (squalène, et tensioactif non ionique hydrophobe) dans deux flacons ou réacteurs. Les deux phases sont chauffées sous agitation à une température légèrement supérieure à la température d'inversion de phase. Les deux phases sont alors introduites dans un mélangeur statique thermostaté au moyen de 2 pompes, dont les débits sont régulés de manière à obtenir la composition de l'émulsion selon l'invention. L'émulsion inverse eau-dans-huile est obtenue durant le passage des deux phases dans le mélangeur statique. L'émulsion inverse est ensuite refroidie par passage en ligne au travers d'un échangeur thermique réfrigéré connecté en sortie du mélangeur statique. L'émulsion eau-dans-huile va alors s'inverser au travers de l'échangeur thermique réfrigéré pour donner lieu à une émulsion huile-dans-eau, qui sera réceptionnée dans un flacon ou réacteur et dont les caractéristiques sont identiques à celles de l'émulsion obtenue par un procédé en lots.

L'émulsion adjuvante selon l'invention est ensuite utilisée pour la préparation d'une composition immunogène. Un mode de réalisation simple consiste à mélanger une solution comprenant au moins un antigène vaccinal avec une émulsion obtenue selon l'un des modes de réalisation qui viennent d'être décrits. La composition immunogène obtenue est sous la forme d'une émulsion huile-dans-eau ou sous la forme d'une émulsion huile-dans-eau thermoréversible lorsque la quantité de squalène représente en masse au moins 5% de la masse totale de la composition immunogène.

Alternativement, il est possible de mélanger l'antigène à la phase aqueuse ou à la phase huileuse avant de préparer l'émulsion. Une telle façon de procéder implique bien sûr qu'il s'agisse d'antigènes qui soient compatibles avec le procédé de thermoinversion.

Les solutions de l'antigène peuvent en outre contenir des sels minéraux et un ou plusieurs tampons, ainsi que tout autre composé habituellement utilisé dans les vaccins, tels que des stabilisants, des conservateurs, ou éventuellement aussi d'autres adjuvants.

Pour la préparation d'une émulsion lyophilisable, on prépare tout d'abord une émulsion concentrée liquide ainsi que cela vient d'être décrit mais en choisissant de préférence comme phase aqueuse de l'eau plutôt qu'une solution tamponnée, puis on dilue cette émulsion par une solution comprenant un alditol, un sucre et un alkylpolyglycoside, par exemple par une solution comprenant du mannitol, du saccharose et du dodécylmaltoside.

L'émulsion obtenue est alors répartie en échantillons (par exemple 0,5ml) et soumise à un cycle de lyophilisation qui peut s'effectuer de la manière suivante :

- chargement des échantillons à +4°C,
- environ 2 heures de congélation à une température de consigne de - 45°C,
- 14 à 19 heures de dessication primaire à une température de consigne de 0°C,
- 3 heures 30 de dessication secondaire à une température de consigne de + 25°C.

L'émulsion obtenue peut alors être conservée en attendant d'être utilisée pour la préparation d'une composition immunogène, c'est-à- dire d'être associée à une composition comprenant des antigènes vaccinaux. Cette étape de la préparation de la composition immunogène peut se faire par reprise de l'émulsion lyophilisée au moyen d'une solution aqueuse comprenant les antigènes. La composition immunogène ainsi obtenue peut ensuite être conservée à l'état liquide, ou être soumise à un nouveau cycle de lyophilisation afin d'être conservée sous forme de lyophilisat, si la nature des antigènes le permet.

De manière alternative, il est possible de diluer directement l'émulsion concentrée par une solution aqueuse comprenant à la fois les antigènes vaccinaux ainsi que l'alditol, le sucre et l'alkylpolyglycoside, et de soumettre ensuite la composition obtenue à la lyophilisation. Une telle façon de procéder implique bien sûr qu'ils s'agissent d'antigènes qui soient compatibles avec un procédé de lyophilisation.

Les exemples qui suivent illustrent différents modes de réalisation de l'invention.

Exemple 1 : Préparation d'une émulsion adjuvant selon l'invention.

On a mélangé dans un bécher 3,71g d'Eumulgin™ Bel et 33,9g d'une solution de mannitol à 10% en tampon PBS, que l'on a homogénéisée sous agitation à environ 30°C. Dans un autre récipient, on a mélangé 2,89g de Dehymuls™ SMO et 19,5g de squalène que l'on a agité magnétiquement.

Lorsque des phases homogènes ont été obtenues dans chacun des récipients, on a incorporé la phase aqueuse à la phase huileuse qui était maintenue sous agitation à 30°C. Lorsque l'incorporation a été terminée, on a chauffé l'émulsion brute obtenue jusqu'à ce que la température atteigne 58-60 °C, tout en maintenant l'agitation.

On a ensuite cessé le chauffage mais on a maintenu l'agitation jusqu'à ce que la température atteigne la température ambiante.

On a alors obtenu une émulsion huile-dans-eau, dont la taille des gouttelettes d'huile était centrée autour de 80 nm (mesure faite au LS230), et dont la composition en masse était la suivante :

- 32,5 % de squalène,
- 6,18 % de polyoxyéthylène (12) cétostéaryéther,
- 4,82 % de monooléate de sorbitanne,
- 6 % de mannitol

Exemple 2 : Composition vaccinale contre le SIDA.

On a préparé des compositions vaccinales comprenant comme antigène une protéine TAT III B détoxifiée. La protéine TAT a été détoxifiée par une réaction d'alkylation en milieu alcalin par utilisation d'iodoacétamide dans les conditions suivantes : nombre de micromoles d'iodoacétamide = 200 X nombre de micromoles de TAT + nombre de micromoles de DTT. Cette protéine détoxifiée et son procédé de préparation sont décrits en détails dans la demande WO99/33346 où elle est identifiée sous le terme de TAT carboxyméthylée. Cet antigène TAT recombinante est conservé en solution, en présence de tampon TRIS 50mM, pH 7,5 à -70°C.

Les compositions vaccinales à administrer ont été préparées à partir de solutions concentrées, afin d'obtenir des doses d'immunisation de 200$\mu$l ayant les compositions quantitatives suivantes :

- pour la composition ayant uniquement l'antigène : 20$\mu$g de TAT en tampon TRIS 50mM, NaCl 100 mM à pH 7,5.
- pour la composition selon l'invention :

  - 20$\mu$g de TAT,
  - 5 mg de squalène,
  - 0,75 mg de Dehymuls™ SMO,
  - 0,94 mg d'Eumulgin™ B1,
  - 0,91 mg de mannitol.

On disposait de 2 groupes de 6 souris BALB/c femelles âgées de 8 semaines, à qui l'on a injecté une des compositions préparées par voie sous-cutanée, à raison d'une dose de 200$\mu$l par souris ; les injections ont été réalisées à J0 et à J21. On a prélevé des échantillons sanguins au sinus retro-orbital à J14 pour l'appréciation de la réponse primaire et à J34 pour la réponse secondaire. La détermination du taux d'IgG1 et d'IgG2a spécifiques a été effectuée grâce à des tests ELISA standardisés.

Les souris ont été sacrifiées à J37 ; on a prélevé leur rate et on a isolé les splénocytes.

Les résultats obtenus en ce qui concerne les réponses humorales sont récapitulés dans le tableau ci-dessous, où les taux d'IgG sont exprimés en unités arbitraires ELISA (log10). Pour chaque groupe de souris, la valeur indiquée dans le tableau est le titre géométrique moyen des valeurs obtenues pour chacune des souris.

| Composition vaccinale | IgG1 à J14 | IgG2a à J14 | IgG1 à J34 | IgG2a à J34 | Rapport IgG1/IgG2a à J34 |
|---|---|---|---|---|---|
| Tat | 2,6 | 1,2 | 4,4 | 3,0 | 25 |
| Tat+ PIT | 3,5 | 2,5 | 5,7 | 5,0 | 5 |

Les résultats obtenus montrent que l'émulsion selon l'invention permet d'accroître globalement la réponse humorale et tend en outre à favoriser la réponse T helper de type 1 car la réponse IgG2a est plus augmentée que la réponse IgG1. En ce qui concerne la réponse cellulaire, on a pu mettre en évidence, par dosage ELISPOT après restimulation avec de la protéine TAT recombinante des splénocytes prélevés, une nette augmentation du nombre de cellules produisant l'interféron $\gamma$ lorsque les splénocytes provenaient de souris immunisées avec une préparation selon l'invention (486 spots par $10^6$ cellules contre 39 par $10^6$ pour la préparation contenant l'antigène seul) . De même, le dosage des cytokines dans les surnageants de culture a montré la plus grande sécrétion à la fois d'interféron $\gamma$ (5028 pg/ml contre 1940 pg/ml) et d'Interleukine 5 (5365 pg/ml contre 2394 pg/ml).

Exemple 3 : Préparation d'une composition vaccinale contre les infections à cytomegalovirus humain.

On a préparé des compositions vaccinales comprenant à titre d'antigène vaccinal une protéine recombinante dérivée d'une glycoprotéine d'enveloppe de la souche Towne du Cytomegalovirus (CMV) dénommée gB dont les séquences nucléotidique et protéique sont décrites dans le brevet USP 5,834,307. Cette protéine recombinante est produite par une lignée CHO recombinante transfectée par un plasmide dénommé pPRgB27clv4 qui contient un gène modifié de la gB. En effet, pour faciliter la production de cette protéine recombinante par la lignée CHO le gène de la gB a été modifié au préalable en supprimant la partie du gène qui code pour la région transmembranaire de la protéine gB correspondant à la séquence d'acides aminés comprise entre la Valine 677 et l'Arginine 752 et en introduisant 3 mutations ponctuelles de sorte que le site de clivage existant dans la gB native a été supprimé. En définitive la protéine recombinante produite par la lignée CHO recombinante correspond à une protéine gB tronquée dépourvue de site de clivage et de région transmembranaire dénommée gBdTM.

La construction du plasmide pPRgB27clv4 et la production de la protéine gB tronquée (gBdTM) par la lignée CHO recombinante sont décrites dans US 6,100,064. La purification de la protéine gB tronquée est réalisée sur colonne chromatographique d'immunoaffinité en utilisant l'anticorps monoclonal 15D8 décrit par Rasmussen L et al. (J. Virol. (1985) 55 : 274-280).

A partir d'une composition stock à 0.975 mg/ml d'antigène gB ainsi obtenu et maintenu en tampon phosphate, de l'émulsion selon l'invention concentrée telle que décrite à l'exemple 1, et d'une émulsion de l'art antérieur obtenue par microfluidisation, on a préparé des doses de compositions immunisantes de $50\mu l$ ayant les compositions suivantes :

- 2 $\mu$g de gB en tampon citrate à pH 6, (groupe appelé gB seule),
- 2$\mu$g de gB ; 1,075mg de squalène ; 0,133 mg de Montane™ VG 85 et 0,125 mg de Tween™80 en tampon citrate à pH 6, (groupe appelé « avec émulsion de l'art antérieur »),
- 2 $\mu$g de gB ; 1,25 mg de squalène ; 0,185 mg de Dehymuls™ SMO ; 0,235 mg d'Eumulgin™ B1 et 0,230 mg de mannitol en tampon PBS à pH 7,4. (groupe appelé « avec émulsion de l'invention »)

On disposait de 3 groupes de 10 souris Outbred OF1 femelles, âgées de 8 semaines que l'on a immunisées 2 fois, à J0 et à J21 par voie sous-cutanée, par une des compositions indiquées ci-dessus (chaque groupe de souris reçoit les 2 fois la même composition) On a prélevé à J20 et J34 au sinus retro-orbital des échantillons sanguins qui ont été utilisés pour déterminer les concentrations en anticorps de type IgG1 et IgG2a spécifiques de l'antigène gB.

Les dosages ont été réalisés grâce à des tests ELISA ; les résultats obtenus sont rassemblés dans le tableau ci-dessous, et sont exprimés en $\log_{10}$ des titres ELISA. Les valeurs indiquées sont les valeurs moyennes obtenues pour chaque groupe de souris.

| Nature des Groupes | J20 | | J34 | | Ratio à J34 IgG1/IgG2a |
|---|---|---|---|---|---|
| | IgG1 | IgG2a | IgG1 | IgG2a | |
| gB seule | 2,474 | 2,094 | 3,801 | 2,941 | 137 |
| gB + émulsion de l'art antérieur | 4,063 | 2,980 | 5,493 | 4,1.85 | 143 |
| gB + émulsion selon l'invention | 4,615 | 3,914 | 5,615 | 4,854 | 14 |

Ces résultats montrent l'efficacité de l'émulsion selon l'invention qui a permis une plus grande induction d'anticorps à la fois ceux de type IgG1 et IgG2a, avec en outre, par rapport à l'émulsion selon l'art antérieur l'obtention du résultat recherché dans le cadre d'un vaccin contre le cytomégalovirus humain, qui est d'orienter la réponse immunitaire vers une réponse TH1 (dont un indicateur est le taux d'IgG2a), tout en maintenant la réponse de type TH2 (dont un indicateur est le taux d'IgG1) à un niveau suffisant.

Exemple 4 : Composition vaccinale contre la grippe.

On disposait d'antigènes de virus grippal obtenus selon le procédé décrit aux exemples de la demande WO9605294, à l'exception du fait que la souche virale utilisée était la souche A/New Caledonia H1N1 .

A partir de cette préparation d'antigènes, de l'émulsion selon l'invention concentrée obtenue à l'exemple 1, et d'une suspension d'aluminium fournie par REHEIS sous la dénomination AlOOH Rehydra, on a préparé des doses d'immunisation de $50\mu l$ qui avaient la composition indiquée ci-après, où les quantités d'antigènes de la grippe sont exprimées en poids d'hémagglutinine HA :

- soit 1 $\mu$g de HA en tampon PBS,
- soit 5 $\mu$g de HA en tampon PBS,
- soit 1 $\mu$g de HA et 60 $\mu$g d'hydroxyde d'aluminium,
- soit 1 $\mu$g de HA ; 1,25 mg de squalène ; 0,185 mg de Dehymuls™ SMO ; 0,235 mg d'Eumulgin™ B1 ; 0,21 mg de mannitol ; le tout en tampon PBS.

On disposait de 8 groupes de 5 souris BALB/c femelles âgées de 8 semaines, à qui on a administré à J0 les compositions préparées selon la répartition suivante :

- un groupe a reçu la composition ayant 1 $\mu$g de HA par voie sous-cutanée,
- un groupe a reçu la même composition ayant 1 $\mu$g de HA par voie intra-dermique,
- un groupe a reçu la composition ayant 5$\mu$g de HA par voie sous-cutanée,
- un groupe a reçu la même composition ayant 5 $\mu$g de HA par voie intra-dermique,
- un groupe a reçu la composition ayant 1 $\mu$g de HA et 60 $\mu$g d'aluminium par voie sous-cutanée,
- un groupe a reçu la même composition ayant 1 $\mu$g de HA et 60 $\mu$g d'aluminium par voie intra-dermique,
- un groupe a reçu la composition ayant 1$\mu$g de HA et l'émulsion selon l'invention par voie sous-cutanée,
- un groupe a reçu la composition ayant 1$\mu$g de HA et l'émulsion selon l'invention par voie intra-dermique.

On a prélevé sur chacune des souris des échantillons sanguins à J14, J28, J41, J56 et J105.
Les sérums des souris immunisées ont été testés d'une part par la technique ELISA afin d'évaluer leur teneur en anticorps totaux induits contre la souche grippale A/H1N1 du vaccin trivalent (Les titres anticorps sont exprimés en unités ELISA arbitraires en valeur log10, avec un seuil de détection à 1,3 log10) et d'autre part par la technique IHA (inhibition d'hémaglutination) pour déterminer leur teneur en anticorps fonctionnels contre la souche grippale A/H1N1. Les titres anticorps sont exprimés en inverse de dilution en valeur arithmétique, avec un seuil de détection à 5.
Les résultats obtenus sont repris dans les tableaux ci-après, dans lesquels les valeurs - indiquées représentent la moyenne des titres des souris de chaque groupe.
Titres ELISA :

| Groupe de souris | Voie d'immunisation | J14 | J28 | J41 | J56 | J105 |
|---|---|---|---|---|---|---|
| HA 1$\mu$g | sous-cutanée | 2,838 | 3,288 | 3,556 | 3,581 | 3,535 |
| HA 5$\mu$g | sous-cutanée | 3,203 | 3,642 | 3,836 | 3,732 | 3,844 |
| HA 1$\mu$g +AlOOH | sous-cutanée | 2,860 | 3,363 | 3,843 | 3,951 | 3,982 |
| HA 1$\mu$g+émulsion | sous-cutanée | 4,043 | 4,511 | 4,753 | 4,723 | 4,681 |
| HA 1$\mu$g | intradermique | 2,174 | 2,814 | 3,143 | 3,075 | 2,735 |
| HA 5$\mu$g | intradermique | 2,839 | 3,297 | 3,496 | 3,549 | 3,479 |
| HA 1$\mu$g +AlOOH | intradermique | 2,654 | 3,004 | 3,137 | 3,020 | 2,724 |
| HA 1$\mu$g +émulsion | intradermique | 4,134 | 4,692 | 4,895 | 4,911 | 4,823 |

Titres IHA :

| Groupe de souris | Voie d'immunisation | J14 | J28 | J41 | J56 | J105 |
|---|---|---|---|---|---|---|
| OHA 1$\mu$g | sous-cutanée | 5 | 6 | 5 | 40 | 23 |
| HA 5$\mu$g | sous-cutanée | | 5 | 9 | 20 | |
| HA 1$\mu$g +AlOOH | sous-cutanée | 5 | 15 | 23 | 121 | 160 |
| HA 1 $\mu$g +émulsion | sous-cutanée | 6 | 160 | 368 | 422 | 485 |
| HA 1$\mu$g | intradermique | 5 | 5 | 5 | 23 | 8 |
| HA 5$\mu$g | intradermique | | 5 | 8 | 10 | |
| HA 1$\mu$g +AlOOH | intradermique | 5 | 5 | 5 | 26 | 7 |
| HA 1$\mu$g +émulsion | intradermique | 7 | 557 | 1640 | 1557 | 1844 |

Ces résultats montrent l'intérêt de la présente invention ; en effet, l'hydroxyde d'aluminium qui est un adjuvant bien connu et largement utilisé dans l'art antérieur n'a pas permis d'augmenter l'immunogénicité des antigènes de la grippe avec la même rapidité et la même force que la formulation selon l'invention ; on constate aussi que la composition selon l'invention a été particulièrement efficace, que ce soit par voie sous-cutanée, ou par voie intradermique.

Exemple 5 : Composition vaccinale contre la grippe.

On a voulu évaluer chez des souris, l'intérêt de la présente invention pour diminuer la quantité d'antigène vaccinal lorsque qu'il s'agit d'un vaccin contre la grippe contenant comme antigènes 3 souches de virus grippal, et qui serait administré par voie intra-dermique.

A cette fin, on a dilué l'émulsion concentrée de l'exemple 1 par du tampon PBS afin d'obtenir une émulsion à 5% de squalène, que l'on a encore diluée pour moitié par une composition comprenant les antigènes.

On disposait en effet d'une composition comprenant du virus grippal provenant de 3 souches virales différentes obtenues de la manière décrite dans la demande de brevet WO9605294, les 3 souches étant ici la souche A/New Caledonia (H1N1), la souche A/Wyoming (H3N2), la souche B/Jiangsu. Une telle composition vaccinale trivalente est une composition classique pour un vaccin contre la grippe et correspond au vaccin commercialisé dans l'hémisphère Nord lors de la campagne grippe 2004. Les quantités d'antigènes de chacune des souches virales sont appréciées par leur quantité d'hémagglutinines HA.

Les doses d'immunisation préparées, de volume 50μl, avaient les compositions indiquées ci-après :

- 0,33 μg de HA de chacune des souches virales en tampon PBS à pH 7,4 ;
- 1,31 μg de HA de chacune des souches virales en tampon PBS à pH 7,4 ;
- 5,25 μg de HA de chacune des souches virales en tampon PBS à pH 7,4 ;
- 10,5 μg de HA de chacune des souches virales en tampon PBS à pH 7,4 ;
- 21 μg de HA de chacune des souches virales en tampon PBS à pH 7,4 ;
- 0,33 μg de HA de chacune des souches virales ; 1,25 mg de squalène ; 0,185 mg de Dehymuls™ SMO ; 0,235 mg d'Eumulgin™ B1 et 0,230 mg de mannitol en tampon PBS à pH 7,4 ;
- 1,31 μg de HA de chacune des souches virales ; 1,25 mg de squalène ; 0,185 mg de Dehymuls™ SMO ; 0,235 mg d'Eumulgin™ B1 et 0,230 mg de mannitol en tampon PBS à pH 7,4 ;
- 5,25 μg de HA de chacune des souches virales ; 1,25 mg de squalène ; 0,185 mg de Dehymuls™ SMO ; 0,235 mg d'Eumulgin™ B1 et 0,230 mg de mannitol en tampon PBS à pH 7,4.

On disposait de 8 groupes de 10 souris BALB/c femelles âgées de 6 à 8 semaines à qui on a administré par voie intradermique (face interne de l'oreille) une des compositions préparées, à raison d'une composition par groupe. 3 semaines après l'immunisation, on a prélevé des échantillons sanguins et on a dosé par ELISA pour chacun des groupes, les IgG induites contre chacune des souches virales ; on a, en outre, effectué, pour chacun des groupes, un test d'inhibition de l'hémagglutinine contre chaque souche virale.

Les résultats obtenus, sont représentés dans le tableau ci-dessous sous forme de moyennes pour chacun des groupes ; les résultats ELISA sont exprimés en $\log_{10}$ d'unités arbitraires, et les résultats IHA sont les titres moyens arithmétiques des inverses de dilution.

| Nature Composition | HIN1 | | H3N2 | | B | |
|---|---|---|---|---|---|---|
| | ELISA | IHA | ELISA | IHA | ELISA | IHA |
| 0,33 μg HA | 3,51 | 35 | 4,38 | 243 | 4,28 | 25 |
| 1,31 μg HA | 3,96 | 65 | 4,74 | 640 | 4,35 | 32 |
| 5,25 μg HA | 4,16 | 92 | 5,05 | 970 | 4,62 | 53 |
| 10,5 μg HA | 4,38 | 197 | 5,18 | 1810 . | 4,71 | 130 |
| 21 μg HA | 4,63 | 260 | 5,37 | 2389 | 4,98 | 184 |
| 0,33 μg HA +émulsion | 4,27 | 226 | 5,20 | 2389 | 4,91 | 149 |
| 1,31 μg HA + émulsion | 4,46 | 279 | 5,37 | 3880 | 4,95 | 171 |
| 5,25 μg HA +émulsion | 4,68 | 394 | 5,58 | 5487 | 5,12 | 22 |

Ces résultats mettent en évidence l'intérêt particulier de l'invention pour réduire la quantité d'antigènes ; en effet, grâce à l'émulsion selon l'invention, on a pu, pour une même réponse immunitaire induite, abaisser de façon très importante la quantité d'antigènes présente dans la dose d'immunisation.

Exemple 6 : Composition vaccinale contre la grippe trivalente dans une population non naïve.

On a voulu tester l'efficacité de l'émulsion de l'invention dans le cas d'un vaccin contre la grippe qui serait administré à des personnes dont l'organisme a déjà été au contact d'antigènes du virus de la grippe, ainsi que cela est fréquemment le cas, soit parce que les personnes ont déjà été en contact avec le virus de la grippe, soit parce qu'elles ont déjà été précédemment vaccinées avec un vaccin anti-grippal.
Selon les informations publiées par C.W. Potter dans Vaccine, 2003, 21 : 940-5, il est possible d'utiliser comme modèle animal pour faire ce test, des souris BALB/c préalablement immunisées par voie intramusculaire par un vaccin trivalent. On a donc préparé des doses d'immunisation de 50 µl comprenant soit uniquement du tampon PBS, soit du vaccin trivalent de la campagne 2004, c'est-à-dire un vaccin comprenant la souche A/New Caledonia (H1N1), la souche A/Wyoming (H3N2),et la souche B/Jiangsu, à raison de 5µg de HA de chacune des souches, en tampon PBS à pH 7,4. On disposait de 6 groupes de 7 souris BALB/c ; on a immunisé 3 groupes avec les doses comprenant uniquement du tampon, et 3 autres avec le vaccin trivalent, par voie intramusculaire.
On a préparé en outre, à partir de l'émulsion concentrée de l'exemple 1, et d'une composition vaccinale comprenant les 3 souches virales de la campagne 2004 mentionnée ci-dessus des doses d'immunisation de 30 µl ayant les compositions suivantes :

-    tampon PBS seul,
-    0,3 µg de HA de chacune des souches virales en tampon PBS,
-    0,3 µg de HA de chacune des souches virales ; 0,75 mg de squalène ; 0,11 mg de Dehymuls™ SMO ; 0,143 mg d'Eumulgin™ B1 et 0,138 mg de mannitol en tampon PBS à pH 7,4.

Chacune des compositions ainsi préparée a été utilisée pour immuniser à J34 par voie intradermique (dans la face interne de l'oreille) à la.fois un groupe de souris ayant reçu au préalable uniquement du tampon PBS, et un groupe de souris ayant reçu une dose de vaccin trivalent.
On a prélevé à J56 un échantillon sanguin de chacune des souris, et on a effectué un dosage des anticorps produits contre la souche H1N1 (A/New Caledonia) grâce à un test d'inhibition de l'hémagglutinine.
Les résultats obtenus ont été résumés dans le tableau ci-après et représentent les valeurs moyennes obtenues pour chaque groupe de souris ayant suivi le même protocole d'immunisation.

| Identification Groupe de souris | Nature de la dose pour le priming im | Nature de la dose pour le boost id | Titre IHA vis-à-vis de la souche H1N1 |
|---|---|---|---|
| A | PBS | PBS | 5 |
| B | PBS | Vaccin trivalent à 0,3 µg HA/souche | 59 |
| C | PBS | Vaccin trivalent à 0,3 µg HA/souche + émulsion | 320 |
| D | Vaccin trivalent à 5 µg HA/souche | PBS | 145 |
| E | Vaccin trivalent à 5 µg HA/souche | Vaccin trivalent à 0,3 µg HA/souche | 476 |
| F | Vaccin trivalent à 5 µg HA/souche | Vaccin trivalent à 0,3 µg HA/souche + émulsion | 861 |

Ces résultats montrent tout l'intérêt de l'invention même chez des individus non naïfs vis-à-vis de l'antigène administré. En effet, contrairement aux observations de C.W.
Potter lors de l'utilisation de ce modèle qui, lui, n'avait pu montrer qu'un faible effet adjuvant des Iscoms lorsqu'ils étaient utilisés pour immuniser des souris pré-infectées ou pré-vaccinées par des antigènes de la grippe, ici on voit que l'émulsion selon l'invention a permis d'augmenter de façon significative la réponse induite, que ce soit avec des souris naïves, ou avec des souris ayant déjà été immunisées par du vaccin grippe.

Exemple 7 : Composition vaccinale trivalente contre la grippe comprenant de faibles

doses d'antigènes

On a préparé à partir de l'émulsion concentrée de l'exemple 1, et d'une composition vaccinale comprenant les 3 souches virales de la campagne 2004 (la souche A/New Caledonia (H1N1), la souche A/Wyoming (H3N2),et la souche B/Jiangsu) des doses d'immunisation de 30 μl ayant les compositions suivantes :

- 0,1 μg de HA de chacune des souches virales en tampon PBS,
- 0,4 μg de HA de chacune des souches virales en tampon PBS,
- 1,6 μg de HA de chacune des souches virales en tampon PBS,
- 6,3 μg de HA de chacune des souches virales en tampon PBS,
- 0,1 μg de HA ; 0,75 mg de squalène ; 0,11 mg de Dehymuls™ SMO ; 0,143 mg d'Eumulgin™ B1 et 0,138 mg de mannitol en tampon PBS à pH 7,4 ;
- 0,4 μg de HA ; 0,75 mg de squalène ; 0,11 mg de Dehymuls™ SMO ; 0,143 mg d'Eumulgin™ B1 et 0,138 mg de mannitol en tampon PBS à pH 7,4.

On disposait de 6 groupes de 8 souris BALB/c femelles âgées de 8 semaines, à qui on a administrée à J0 par voie intradermique (face interne de l'oreille) une dose de 30 μl d'une des compositions indiquées ci-dessus (1 composition par groupe).
Dans chaque groupe, on a administré à nouveau par voie intradermique à la moitié des souris à J29, une 2nde dose ayant la même nature que la 1ère dose administrée.
On a prélevé des échantillons sanguins à J22 et à J43 afin de déterminer les quantités d'anticorps induits.
Les dosages d'anticorps ont été effectués par ELISA pour les anticorps induits à J22 et à J43, vis-à-vis de l'ensemble des souches administrées : H1N1, H3N2 et B, et par IHA vis-à-vis de la souche H1N1 uniquement à la fois à J22, et à J43. Les résultats obtenus ont été résumés dans le tableau ci-après, où les titres exprimés sont les moyennes obtenues pour chaque groupe de souris. En ce qui concerne les résultats à J43, les moyennes ont été réalisées séparément à l'intérieur d'un même groupe, pour les souris ayant reçu 2 doses de vaccin et celles n'en ayant reçu qu'une.

| Composition vaccinale | Titre ELISA H1N1 à J22 | Titre ELISA H3N2 à J22 | Titre ELISA B à J22 | Titre IHA H1N1 à J22 | Titre IHA H1N1 à J43 | |
|---|---|---|---|---|---|---|
| | | | | | Souris Boostées | Souris non boostées |
| 0,1 μg HA | 3,467 | 4,131 | 4,063 | 57 | 95 | 80 |
| 0,4 μg HA | 3,816 | 4,527 | 4,313 | 73 | 226 | 80 |
| 1,6 μg HA | 4,069 | 4,831 | 4,750 | 147 | 1280 | 135 |
| 6,3 μg HA | 4,534 | 5,298 | 4,947 | 320 | 1522 | 320 |
| 0,1 μg HA + émulsion | 4,200 | 5,015 | 4,807 | 207 | 2153 | 538 |
| 0,4 μg HA + émulsion | 4,612 | 5,482 | 5,001 | 453 | 2560 | 640 |

| Composition vaccinale | Titre ELISA H1N1 à J43 | | Titre ELISA H3N2 à J43 | | Titre ELISA B à J43 | |
|---|---|---|---|---|---|---|
| | Souris Boostées | Souris non boostées | Souris Boostées | Souris non boostées | Souris Boostées | Souris non boostées |
| 0,1 μg HA | 3,833 | 3,425 | 4,782 | 4,174 | 4,479 | 3,911 |
| 0,4 μg HA | 4,385 | 3,599 | 5,250 | 4,354 | 5,053 | 4,079 |
| 1,6 μg HA | 4,906 | 3,876 | 5,526 | 4,779 | 5,458 | 4,487 |
| 6,3 μg HA | 5,287 | 4,176 | 6,073 | 5,033 | 5,678 | 4,773 |
| 0,1 μg HA + émulsion | 5,210 | 4,523 | 5,990 | 5,264 | 5,723 | 4,943 |

(suite)

| Composition vaccinale | Titre ELISA H1N1 à J43 | | Titre ELISA H3N2 à J43 | | Titre ELISA B à J43 | |
|---|---|---|---|---|---|---|
| | Souris Boostées | Souris non boostées | Souris Boostées | Souris non boostées | Souris Boostées | Souris non boostées |
| 0,4 µg HA + émulsion | 5,394 | 4,790 | 6,171 | 5,640 | 5,962 | 5,144 |

Ces résultats montrent que grâce à l'émulsion selon l'invention, même avec de faibles doses d'antigènes, on a obtenu des réponses humorales très importantes. Ainsi, on peut noté que les meilleurs résultats sont ceux obtenus avec une dose de 0,4 µg de HA de chacune des souches virales et une émulsion selon l'invention ; ces résultats sont de façon très surprenante, bien meilleurs que ceux obtenus en utilisant une dose de 6,3 µg de HA seule. En outre, on remarque que même chez les individus n'ayant pas reçu de dose de rappel, le système immunitaire continue à induire des anticorps alors que cela n'est pas le cas pour les individus ayant reçu les antigènes vaccinaux non adjuvés.

Exemple 8 : Composition vaccinale contre à grippe trivalente

On a préparé à partir de l'émulsion concentrée de l'exemple 1, et d'une composition vaccinale comprenant les 3 souches virales de la campagne 2004 (la souche A/New Caledonia (H1N1), la souche A/Wyoming (H3N2),et la souche B/Jiangsu) des doses d'immunisation de 30 µl ayant les compositions suivantes :

- 0,1 µg de HA de chacune des souches virales en tampon PBS,
- 0,4 µg de HA de chacune des souches virales en tampon PBS,
- 1,6 µg de HA de chacune des souches virales en tampon PBS,
- 6,3 µg de HA de chacune des souches virales en tampon PBS,
- 0,1 µg de HA ; 0,75 mg de squalène ; 0,11 mg de Dehymuls™ SMO ; 0,143 mg d'Eumulgin™ B 1 et 0,13 8 mg de mannitol en tampon PBS à pH 7,4,
- 0,4 µg de HA ; 0,75 mg de squalène ; 0,11 mg de Dehymuls™ SMO ; 0,143 mg d'Eumulgin™ B1 et 0,13.8 mg de mannitol en tampon PBS à pH 7,4.

On disposait de 6 groupes de 8 souris C57BL/6J femelles âgées de 8 semaines, à qui on a administré à J0 par voie intradermique (face interne de l'oreille) une dose de 30 µl d'une des compositions indiquées ci-dessus (1 composition par groupe).

On a prélevé des échantillons sanguins à J23 afin de déterminer les quantités d'anticorps induits.
Les dosages d'anticorps ont été effectués par ELISA et par IHA pour les anticorps induits vis-à-vis de l'ensemble des souches administrées : H1N1, H3N2 et B.
Les résultats obtenus ont été résumés dans le tableau ci-après, où les titres exprimés sont les moyennes obtenues pour chaque groupe de souris.

| Composition | ELISA H1N1 | ELISA H3N2 | ELISA B | IHA H1N1 | IHA H3N2 | IHA B |
|---|---|---|---|---|---|---|
| 0,1 µg HA | 2,924 | 3,506 | 3,920 | 26 | 174 | 95 |
| 0,4 µg HA | 3,673 | 4,227 | 4,431 | 135 | 269 | 147 |
| 1,6 µg HA | 3,948 | 4,593 | 4,786 | 160 | 381 | 190 |
| 6,3 µg HA | 4,446 | 5,106 | 5,190 | 587 | 1174 | 453 |
| 0,1 µg HA + émulsion | 4,456 | 5,192 | 5,064 | 349 | 1974 | 320 |
| 0,4 µg HA + émulsion | 4,659 | 5,337 | 5,174 | 761 | 2348 | 494 |

A nouveau, les résultats obtenus montrent tout l'intérêt de l'émulsion selon l'invention grâce à laquelle il est possible de réduire très fortement les quantités d'antigènes présents: En effet, on peut globalement considérer qu'avec uniquement 0,1 µg de HA adjuvé par l'émulsion selon l'invention, on obtient d'aussi bons résultats qu'avec une quantités de 6,3 µg de HA.

Exemple 9 : Composition vaccinale trivalente contre la grippe comprenant une émulsion selon l'invention ou selon l'art antérieur.

On a préparé à partir de l'émulsion concentrée de l'exemple 1, et d'une composition vaccinale comprenant les 3 souches virales de la campagne 2004 (la souche A/New Caledonia (H1N1), la souche A/Wyoming (H3N2),et la souche B/Jiangsu) des doses d'immunisation de 30 µl ayant les compositions suivantes :

- 0,3 µg de HA de chacune des souches virales en tampon PBS,
- 6,3 µg de HA de chacune des souches virales en tampon PBS,
- 0,3 µg de HA ; 0,21 mg de squalène 0,031 mg de Dehymuls™ SMO ; 0,040 mg d'Eumulgin™ B1 et 0,039 mg de mannitol en tampon PBS à pH 7,4 (émulsion à 0,7%),
- 0,3 µg de HA ; 0,75 mg de squalène ; 0,11 mg de Dehymuls™ SMO ; 0,143 mg d'Eumulgin™ B1 et 0,138 mg de mannitol en tampon PBS à pH 7,4 (émulsion à 2,5%)
- 0,3 µg de HA ; 0,645 mg de squalène ; 0,075mg de Tween™ 80 ; 0,075mg de Span™ 85 (émulsion selon l'art antérieur obtenue par microfluidisation).

On disposait de 5 groupes de 8 souris BALB/c femelles âgées de 8 semaines, à qui on a administré à J0 par voie intradermique (face interne de l'oreille) une dose de 30 µl d'une des compositions indiquées ci-dessus (1 composition par groupe).

Pour évaluer la quantité d'anticorps induits, on a effectué des prélèvements sanguins à J21 sur lesquels on a déterminé par IHA (inhibition de l'hémagglutination) l'activité contre la souche A/H1N1, la souche A/H3N2, la souche B.

Les résultats obtenus pour chaque groupe de souris sont représentés dans le tableau ci-après.

| Composition testée | IHA contre H1N1 | IHA contre H3N2 | IHA contre B |
|---|---|---|---|
| 0,3 µg HA | 26 | 174 | 8 |
| 6,3 µg HA | 247 | 905 | 73 |
| 0,3 µg HA + émulsion invention à 0,7% | 95 | 640 | 37 |
| 0,3 µg HA + émulsion invention à 2,5% | 269 | 1974 | 73 |
| 0,3 µg HA + émulsion art antérieur | 135 | 987 | 57 |

Ces résultats montrent qu'avec une émulsion obtenue selon l'invention grâce à un procédé de préparation très simple d'inversion de phase au moyen d'un changement de température, on a obtenu un adjuvant qui est aussi bon, et même légèrement meilleur que l'émulsion de l'art antérieur obtenue en utilisant des taux de cisaillement très importants.

Exemple 10 : Composition vaccinale trivalente contre la grippe comprenant une émulsion selon l'invention à différentes concentrations

On a préparé à partir de l'émulsion concentrée de l'exemple 1, et d'une composition vaccinale comprenant les 3 souches virales de la campagne 2004 (la souche A/New Caledonia (H1N1), la souche A/Wyoming (H3N2),et la souche B/Jiangsu) des doses d'immunisation de 30 µl ayant les compositions suivantes :

- 0,3 µg de HA de chacune des souches virales en tampon PBS,
- 6,3 µg de HA de chacune des souches virales en tampon PBS,
- 0,3 µg de HA de chacune des souches virales ; 0,12 mg de squalène ; 0,018 mg de Dehymuls™ SMO ; 0,023 mg d'Eumulgin™ B1 et 0,022 mg de mannitol en tampon PBS à pH 7,4 (émulsion à 0,4%),
- 0,3 µg de HA de chacune des souches virales ; 0,299 mg de squalène ; 0,044 mg de Dehymuls™ SMO ; 0,057 mg d'Eumulgin™ B 1 et 0,055 mg de mannitol en tampon PBS à pH 7,4 (émulsion à 1%)
- 0,3 µg de HA de chacune des souches virales ; 0,75 mg de squalène ; 0,11 mg de Dehymuls™ SMO ; 0,143 mg d'Eumulgin™ B1 et 0,138 mg de mannitol en tampon PBS à pH 7,4 (émulsion à 2,5%)

On disposait de 5 groupes de 8 souris BALB/c femelles âgées de 8 semaines, à qui on a administré à J0 par voie intradermique (face interne de l'oreille) une dose de 30 µl d'une des compositions indiquées ci-dessus (1 composition par groupe).

Pour évaluer la quantité d'anticorps induits, on a effectué des prélèvements sanguins à J21 sur lesquels on a déterminé par ELISA les anticorps anti-H1N1, les anticorps anti-H3N2 et les anticorps anti-B, et par IHA (inhibition de l'hémagglutination) l'activité contre la souche A/H1N1, la souche A/H3N2, la souche B.

Les résultats obtenus sont représentés dans le tableau ci-dessous sous forme de moyennes pour chacun des groupes ; les résultats ELISA sont exprimés en $\log_{10}$ d'unités arbitraires ELISA et les résultats IHA sont les titres moyens arithmétiques des inverses de dilution.

| Composition testée | Anti-H1N1 | | Anti-H3N2 | | Anti-B | |
|---|---|---|---|---|---|---|
| | ELISA | IHA | ELISA | IHA | ELISA | IHA |
| 0,3 µg HA | 3,864 | 67 | 4,594 | 320 | 4,406 | 31 |
| 6,3 µg HA | 4,478 | 269 | 5,041 | 1660 | 5,053 | 135 |
| 0,3 µg HA + émulsion invention 0,4% | 4,053 | 108 | 4,827 | 525 | 4,545 | 54 |
| 0,3 µg HA + émulsion invention à 1 % | 4,312 | 269 | 5,074 | 1174 | 4,733 | 123 |
| 0,3 µg HA + émulsion invention à 2,5% | 4,425 | 293 | 5,200 | 1974 | 4,840 | 123 |

Ces résultats confirment à nouveau que, quelle que soit la souche évaluée, l'émulsion selon l'invention a permis, avec une dose très faible d'antigènes, d'avoir une réponse du système immunitaire très importante.

Exemple 11 : Préparation d'une composition lyophilisable

On a procédé comme à l'exemple 1, mais en utilisant de l'eau au lieu du tampon ; l'émulsion obtenue a ensuite été diluée avec une solution aqueuse comprenant du mannitol, du saccharose et du dodécylmaltoside, afin d'obtenir une émulsion dont la composition finale était la suivante :

- 5% de squalène,
- 0,95% de polyoxyéthylène cétostéaryl éther,
- 0,75% de monooléate de sorbitanne
- 3% de mannitol,
- 2% de dodécylmaltoside,
- 6% de saccharose

Cette émulsion a été lyophilisée et conservée 3 mois à 4°C ; puis après reconstitution, on a constaté que ses propriétés étaient conservées, notamment ses qualités d'émulsion monodisperse, avec des valeurs de d50 et de d90 proches de celles mesurées avant lyophilisation.

Cette émulsion a pu être diluée au ½ avec une solution comprenant des antigènes vaccinaux afin d'obtenir une composition vaccinale.

Exemple 12 : Comparaison de l'effet adjuvant de l'émulsion selon l'invention et d'un tensioactif présent dans l'émulsion.

On a voulu évaluer l'activité adjuvante de l'émulsion selon l'invention, comparativement à celle du tensioactif Eumulgin™ B1 qui est présent dans l'émulsion. Pour cela, on a effectué un test sur souris au moyen des antigènes de la grippe.

A cette fin, on disposait d'antigènes de virus grippal obtenus selon le procédé décrit aux exemples de la demande WO9605294, à l'exception du fait que la souche virale utilisée était la souche A/New Caledonia H1N1. On disposait également d'une composition vaccinale comprenant les 3 souches virales de la campagne 2004 (la souche A/New Caledonia (H1N1), la souche A/Wyoming (H3N2), et la souche B/Jiangsu).

On a préparé à partir de l'émulsion concentrée obtenue selon l'invention et décrite à l'exemple 1, d'Eumulgin™B1, et des compositions d'antigènes de virus de la grippe, des doses d'immunisation de 100µl qui avaient les compositions suivantes :

- 1 µg de HA de la souche H1N1 en tampon PBS à pH 7,4
- 5µg de HA de la souche H1N1 en tampon PBS à pH 7,4
- 1µg de HA de la souche H1N1 ; 2,5mg de squalène ; 0,37 mg de Dehymuls™ SMO ; 0,48 mg d'Eumulgin™ B1 et 0,46 mg de mannitol en tampon PBS à pH 7,4 ;
- 1µg de HA de la souche H1N1 et 0,48 mg d'Eumulgin™ B1 en tampon PBS à pH 7,4 ;
- 0,33 µg de HA de chacune des souches virales en tampon PBS à pH 7,4

- 1,66 μg de HA de chacune des souches virales en tampon PBS à pH 7,4
- 0,33 μg de HA de chacune des souches virales ; 2,5mg de squalène ; 0,37 mg de Dehymuls™ SMO ; 0,48 mg d'Eumulgin™ B1 et 0,46 mg de mannitol en tampon PBS à pH 7,4 ;
- 0,33 μg de HA de chacune des souches virales et 0,48 mg d'Eumulgin™ B1 en tampon PBS à pH 7,4.

On disposait de 8 groupes de 8 souris BALB/c femelles que l'on a immunisées en intramusculaire par une seule injection à J0. On a prélevé à J21 et J35 des échantillons sanguins pour évaluer par un dosage ELISA leur teneur en anticorps totaux induits contre la souche grippale A/H1N1 ou contre chacune des souches du vaccin trivalent. Les titres anticorps, indiqués dans le tableau ci-après, sont exprimés en unités ELISA arbitraires en valeur log10, avec un seuil de détection à 1,3 log10.

|  | Titres à J21 | | | Titres à J35 | | |
|---|---|---|---|---|---|---|
| Compositions des doses d'immunisation | H1N1 | H3N2 | B | H1N1 | H3N2 | B |
| H1N1 à 1 μg | 2,745 | | | 2,848 | | |
| H1N1 à 5 μg | 3,057 | | | 3,139 | | |
| H1N1 à 1 μg + émulsion invention | 4,002 | | | 4,128 | | |
| H1N1 à 1 μg + Eumulgin™B1 | 3,019 | | | 3,119 | | |
| Vaccin trivalent à 1 μg de HA total | 3,111 | 3,718 | 3,706 | 3,218 | 4,147 | 3,935 |
| Vaccin trivalent à 5 μg de HA total | 3,692 | 4,386 | 3,975 | 3,776 | 4,632 | 4,225 |
| Vaccin trivalent à 1 μg de HA total + émulsion invention | 4,252 | 5,002 | 4,596 | 4,186 | 5,214 | 4,897 |
| Vaccin trivalent à 1 μg de HA total + Eumulgin™B1 | 3,146 | 3,712 | 3,950 | 3,241 | 4,177 | 4,242 |

Les résultats obtenus dans ce test confirment ce qui a déjà été obtenu dans des tests précédents, à savoir que l'émulsion selon l'invention permet de réduire fortement la dose d'antigènes pour une même réponse du système immunitaire ; on a en effet obtenu une meilleure réponse en utilisant l'émulsion selon l'invention et seulement 1 μg de HA plutôt qu'en utilisant une dose de 5 μg de HA sans adjuvant.
On observe en outre que le tensioactif utilisé ne présente pas vraiment d'effet adjuvant lorsqu'il est utilisé seul, alors que l'émulsion selon l'invention est elle fortement adjuvante vis-à-vis de toutes les souches testées.

**Revendications**

1.  Emulsion adjuvante huile-dans-eau **caractérisée en ce qu'**elle comprend au moins :

     - du squalène,
     - un solvant aqueux,
     - un tensioactif non-ionique qui est un polyoxyéthylène alkyl éther dont la balance hydrophile/lipophile (HLB) est supérieure ou égale à 10,
     - un tensioactif non ionique hydrophobe dont la HLB est inférieure à 9,

    **en ce qu'**elle est thermoréversible, et **en ce que** 90% de la population volumique des gouttes d'huile a une taille inférieure à 200 nm.

2.  Emulsion selon la revendication précédente, **caractérisée en ce que** 90% de la population volumique des gouttes d'huile a une taille inférieure à 160 nm.

3.  Emulsion selon une des revendications précédentes, **caractérisée en ce que** 90% de la population volumique des gouttes d'huile a une taille inférieure à 150 nm.

4.  Emulsion selon une des revendications précédentes, **caractérisée en ce que** 50% de la population volumique des gouttes d'huile a une taille inférieure à 100 nm.

5.  Emulsion selon une des revendications précédentes, **caractérisée en ce que** 50% de la population volumique des gouttes d'huile a une taille inférieure à 90 nm.

**6.** Emulsion selon une des revendications précédentes, **caractérisée en ce qu'**elle comprend en outre au moins un alditol.

**7.** Emulsion selon la revendication 1, **caractérisée en ce que** le tensioactif non ionique hydrophobe comprend un ester de sorbitanne ou un ester de mannide.

**8.** Emulsion selon une des revendications précédentes, **caractérisée en ce que** le polyoxyéthylène alkyl éther est le polyoxyéthylène(12) cétostéaryléther.

**9.** Emulsion selon une des revendications précédentes, **caractérisée en ce que** l'alditol est choisi parmi le glycérol, l'érythritol, le xylitol, le sorbitol, le mannitol.

**10.** Emulsion selon une des revendications précédentes, **caractérisée en ce que** le tensioactif non ionique hydrophobe est le monooléate de sorbitanne.

**11.** Emulsion selon une des revendications précédentes, **caractérisée en ce que** la quantité de squalène est comprise entre 5 et 45%.

**12.** Emulsion selon une des revendications précédentes, **caractérisée en ce que** la quantité de tensioactif à base de polyoxyéthylène alkyl éther est comprise entre 0,9 et 9%.

**13.** Emulsion selon une des revendications précédentes, **caractérisée en ce que** la quantité de tensioactif non ionique hydrophobe est comprise entre 0,7 et 7%.

**14.** Emulsion adjuvante selon une des revendications précédentes, **caractérisée en ce qu'**elle comprend :

- 32,5 % de squalène,
- 6,18 % de polyoxyéthylène (12) cétostéaryéther,
- 4,82 % de monooléate de sorbitanne,
- 6 % de mannitol.

**15.** Emulsion selon une des revendications précédentes, **caractérisée en ce qu'**elle comprend en outre un alkylpolyglycoside.

**16.** Emulsion selon une des revendications précédentes, **caractérisée en ce qu'**elle comprend en outre un cryoprotecteur.

**17.** Utilisation d'une émulsion selon une des revendications précédentes pour la préparation d'une composition immunogène destinée à être administrée par voie intramusculaire.

**18.** Utilisation d'une émulsion selon une des revendications 1 à 16, pour la préparation d'une composition immunogène destinée à être administrée par voie intra-dermique.

**19.** Utilisation d'une émulsion selon une des revendications 1 à 16, pour la préparation d'une composition immunogène destinée à être administrée par voie sous-cutanée.

**20.** Utilisation d'une émulsion selon une des revendications 1 à 16, pour la préparation d'une composition immunogène destinée à être administrée contre la grippe.

**21.** Utilisation d'une émulsion selon une des revendications 1 à 16, pour la préparation d'une composition immunogène destinée à être administrée contre le SIDA.

**22.** Utilisation d'une émulsion selon une des revendications 1 à 16, pour la préparation d'une composition immunogène destinée à être administrée contre les pathologies à Cytomegalovirus humain.

**23.** Procédé de préparation d'une composition immunogène comprenant au moins un antigène vaccinal et une émulsion huile-dans-eau, **caractérisé en ce que** l'huile est du squalène et **en ce que** l'émulsion huile-dans-eau est obtenue par un procédé d'inversion de phase par variation de la température.

24. Procédé selon la revendication précédente, **caractérisé en ce qu'**il comprend au moins une étape de préparation de l'émulsion huile-dans-eau par refroidissement d'une émulsion inverse eau-dans-huile qui comprend au moins :

- du squalène,
- un solvant aqueux,
- un tensioactif non ionique hydrophile qui est un polyoxyéthylène alkyl éther dont la balance hydrophile/lipophile (HLB) est supérieure ou égale à 10,
- un tensioactif non ionique hydrophobe dont la HLB est inférieure à 9.

25. Procédé selon la revendication précédente, **caractérisé en ce qu'**on obtient l'émulsion inverse eau-dans-huile en mélangeant du squalène, un solvant aqueux, un tensioactif non ionique qui est un polyoxyéthylène alkyl éther dont la balance hydrophile/lipophile (HLB) est supérieure ou égale à 10, et un tensioactif non ionique hydrophobe dont la HLB est inférieure à 9 afin d'obtenir tout d'abord une émulsion grossière huile-dans-eau, et on chauffe ensuite cette émulsion jusqu'à au moins la température d'inversion de phase afin d'obtenir l'émulsion inverse.

26. Procédé selon la revendication 24, selon lequel :

- on chauffe, séparément, à une température au moins égale à la température d'inversion de phase, d'une part une phase aqueuse comprenant un solvant aqueux et un tensioactif qui est un polyoxyéthylène alkyl éther dont la balance hydrophile/lipophile (HLB) est supérieure ou égale à 10 et, d'autre part, une phase huileuse comprenant du squalène et un tensioactif hydrophobe dont la HLB est inférieure à 9 puis,
- on mélange les 2 phases d'obtenir une émulsion inverse eau-dans-huile.

27. Procédé selon la revendication 24, selon lequel:

- on chauffe, séparément, à une température inférieure à la température d'inversion de phase de l'émulsion d'une part une phase aqueuse comprenant un solvant aqueux et un tensioactif qui est un polyoxyéthylène alkyl éther dont la balance hydrophile/lipophile (HLB) est supérieure ou égale à 10 et, d'autre part, une phase huileuse comprenant du squalène et un tensioactif hydrophobe dont la HLB est inférieure à 9,
- puis on mélange les 2 phases afin d'obtenir une émulsion huile-dans-eau,
- puis on chauffe l'émulsion huile-dans-eau obtenue à une température au moins égale à la température d'inversion de phase, afin d'obtenir une émulsion inverse eau-dans-huile.

28. Procédé selon une des revendications 23 à 27, **caractérisé en ce que** la température d'inversion de phase est comprise entre 45 et 80 °C.

29. Procédé selon la revendication précédente, **caractérisé en ce que** la température d'inversion de phase comprise entre 50 et 65°C.

30. Procédé selon une des revendications 23 à 29, **caractérisé en ce qu'**il comprend en outre au moins une étape de lyophilisation.

31. Composition immunogène **caractérisée en ce qu'**elle peut être obtenue par le procédé selon une des revendications 23 à 30.

**Claims**

1. Oil-in-water adjuvant emulsion **characterized in that** it comprises at least:

- squalene,
- an aqueous solvent,
- a nonionic surfactant which is a polyoxyethylene alkyl ether for which the hydrophilic/lipophilic balance (HLB) is greater than or equal to 10,
- a hydrophobic nonionic surfactant for which the HLB is less than 9,

**in that** it is thermoreversible and **in that** 90% of the population by volume of the oil drops has a size less than 200 nm.

2. Emulsion according to the preceding claim, **characterized in that** 90% of the population by volume of the oil drops has a size less than 160 nm.

3. Emulsion according to one of the preceding claims, **characterized in that** 90% of the population by volume of the oil drops has a size less than 150 nm.

4. Emulsion according to one of the preceding claims, **characterized in that** 50% of the population by volume of the oil drops has a size less than 100 nm.

5. Emulsion according to one of the preceding claims, **characterized in that** 50% of the population by volume of the oil drops has a size less than 90 nm.

6. Emulsion according to one of the preceding claims, **characterized in that** it also comprises at least one alditol.

7. Emulsion according to Claim 1, **characterized in that** the hydrophobic nonionic surfactant comprises a sorbitan ester or a mannide ester.

8. Emulsion according to one of the preceding claims, **characterized in that** the polyoxyethylene alkyl ether is polyoxyethylene(12) cetostearyl ether.

9. Emulsion according to one of the preceding claims, **characterized in that** the alditol is chosen from glycerol, erythritol, xylitol, sorbitol and mannitol.

10. Emulsion according to one of the preceding claims, **characterized in that** the hydrophobic nonionic surfactant is sorbitan monooleate.

11. Emulsion according to one of the preceding claims, **characterized in that** the amount of squalene is between 5 and 45%.

12. Emulsion according to one of the preceding claims, **characterized in that** the amount of polyoxyethylene alkyl ether-based surfactant is between 0.9 and 9%.

13. Emulsion according to one of the preceding claims, **characterized in that** the amount of hydrophobic nonionic surfactant is between 0.7 and 7%.

14. Adjuvant emulsion according to one of the preceding claims, **characterized in that** it comprises:

- 32.5% of squalene,
- 6.18% of polyoxyethylene(12) cetostearyl ether,
- 4.82% of sorbitan monooleate,
- 6% of mannitol.

15. Emulsion according to one of the preceding claims, **characterized in that** it also comprises an alkylpolyglycoside.

16. Emulsion according to one of the preceding claims, **characterized in that** it also comprises a cryoprotective agent.

17. Use of an emulsion according to one of the preceding claims, for preparing an immunogenic composition intended to be administered intramuscularly.

18. Use of an emulsion according to one of Claims 1 to 16, for preparing an immunogenic composition intended to be administered intradermally.

19. Use of an emulsion according to one of Claims 1 to 16, for preparing an immunogenic composition intended to be administered subcutaneously.

20. Use of an emulsion according to one of Claims 1 to 16, for preparing an immunogenic composition intended to be administered against the flu.

21. Use of an emulsion according to one of Claims 1 to 16, for preparing an immunogenic composition intended to be administered against AIDS.

22. Use of an emulsion according to one of Claims 1 to 16, for preparing an immunogenic composition intended to be administered against human cytomegalovirus pathologies.

23. Process for preparing an immunogenic composition comprising at least one vaccine antigen and an oil-in-water emulsion, **characterized in that** the oil is squalene and **in that** the oil-in-water emulsion is obtained by means of a temperature-variation phase-inversion process.

24. Process according to the preceding claim, **characterized in that** it comprises at least one step for preparing the oil-in-water emulsion by cooling a water-in-oil inverse emulsion, which comprises at least:

   - squalene,
   - an aqueous solvent,
   - a hydrophilic nonionic surfactant which is a polyoxyethylene alkyl ether for which the hydrophilic/lipophilic balance (HLB) is greater than or equal to 10,
   - a hydrophobic nonionic surfactant for which the HLB is less than 9.

25. Process according to the preceding claim, **characterized in that** the water-in-oil inverse emulsion is obtained by mixing squalene, an aqueous solvent, a nonionic surfactant which is a polyoxyethylene alkyl ether for which the hydrophilic/lipophilic balance (HLB) is greater than or equal to 10, and a hydrophobic nonionic surfactant for which the HLB is less than 9 so as to obtain, first of all, an oil-in-water coarse emulsion, and this emulsion is then heated to at least the phase-inversion temperature so as to obtain the inverse emulsion.

26. Process according to Claim 24, according to which:

   - firstly, an aqueous phase comprising an aqueous solvent and a surfactant which is a polyoxyethylene alkyl ether for which the hydrophilic/lipophilic balance (HLB) is greater than or equal to 10 and, secondly, an oily phase comprising squalene and a hydrophobic surfactant for which the HLB is less than 9 are heated, separately, to a temperature at least equal to the phase-inversion temperature, and then
   - the 2 phases are mixed so as to obtain a water-in-oil inverse emulsion.

27. Process according to Claim 24, according to which:

   - firstly, an aqueous phase comprising an aqueous solvent and a surfactant which is a polyoxyethylene alkyl ether for which the hydrophilic/lipophilic balance (HLB) is greater than or equal to 10 and, secondly, an oily phase comprising squalene and a hydrophobic surfactant for which the HLB is less than 9 are heated, separately, to a temperature below the phase-inversion temperature of the emulsion,
   - the 2 phases are then mixed so as to obtain an oil-in-water emulsion,
   - the oil-in-water emulsion obtained is then heated to a temperature at least equal to the phase-inversion temperature so as to obtain a water-in-oil inverse emulsion.

28. Process according to one of Claims 23 to 27, **characterized in that** the phase-inversion temperature is between 45 and 80°C.

29. Process according to the preceding claim, **characterized in that** the phase-inversion temperature is between 50 and 65°C.

30. Process according to one of Claims 23 to 29, **characterized in that** it also comprises at least one lyophilization step.

31. Immunogenic composition, **characterized in that** it can be obtained by means of the process according to one of Claims 23 to 30.

**Patentansprüche**

1. Adjuvans-Emulsion Öl-in-Wasser, **dadurch gekennzeichnet, dass** sie mindestens umfasst:

   - Squalen,
   - ein wässeriges Lösungsmittel,
   - ein nichtionisches oberflächenaktives Mittel, das ein Polyoxyethylen-alkylether ist, dessen Gleichgewicht hydrophil/ lipophil (HLB) größer als oder gleich 10 ist,
   - ein hydrophobes nichtionisches oberflächenaktives Mittel, dessen HLB niedriger als 9 ist,

   und **dadurch**, dass sie thermoreversibel ist, und **dadurch**, dass 90 % der volumenmäßigen Population der Öltröpfchen eine Größe von unter 200 nm besitzen.

2. Emulsion nach dem vorstehenden Anspruch, **dadurch gekennzeichnet, dass** 90 % der volumenmäßigen Population der Öltröpfchen eine Größe von unter 160 nm besitzen.

3. Emulsion nach einem der vorstehenden Ansprüche, **dadurch gekennzeichnet, dass** 90 % der volumenmäßigen Population der Öltröpfchen eine Größe von unter 150 nm besitzen.

4. Emulsion nach einem der vorstehenden Ansprüche, **dadurch gekennzeichnet, dass** 50 % der volumenmäßigen Population der Öltröpfchen eine Größe von unter 100 nm besitzen.

5. Emulsion nach einem der vorstehenden Ansprüche, **dadurch gekennzeichnet, dass** 50 % der volumenmäßigen Population der Öltröpfchen eine Größe von unter 90 nm besitzen.

6. Emulsion nach einem der vorstehenden Ansprüche, **dadurch gekennzeichnet, dass** sie außerdem mindestens ein Alditol umfasst.

7. Emulsion nach Anspruch 1, **dadurch gekennzeichnet, dass** das hydrophobe nichtionische oberflächenaktive Mittel einen Ester von Sorbitan oder einen Ester von Mannid umfasst.

8. Emulsion nach einem der vorstehenden Ansprüche, **dadurch gekennzeichnet, dass** der Polyoxyethylen-alkylether der Polyoxyethylen-(12)cetostearylether ist.

9. Emulsion nach einem der vorstehenden Ansprüche, **dadurch gekennzeichnet, dass** das Alditol ausgewählt wird unter Glycerol, Erythritol, Xylitol, Sorbitol, Mannitol.

10. Emulsion nach einem der vorstehenden Ansprüche, **dadurch gekennzeichnet, dass** das hydrophobe nichtionische oberflächenaktive Mittel das Sorbitan-monooleat ist.

11. Emulsion nach einem der vorstehenden Ansprüche, **dadurch gekennzeichnet, dass** die Menge an Squalen zwischen einschließlich 5 und 45 % beträgt.

12. Emulsion nach einem der vorstehenden Ansprüche, **dadurch gekennzeichnet, dass** die Menge an oberflächenaktivem Mittel auf der Basis von Polyoxyethylen-alkylether zwischen einschließlich 0,9 und 9 % beträgt.

13. Emulsion nach einem der vorstehenden Ansprüche, **dadurch gekennzeichnet, dass** die Menge an hydrophobem nichtionischen oberflächenaktiven Mittel zwischen einschließlich 0,7 und 7 % beträgt.

14. Adjuvans-Emulsion nach einem der vorstehenden Ansprüche, **dadurch gekennzeichnet, dass** sie umfasst:

   • 32,5 % Squalen,
   • 6,18 % Polyoxyethylen-(12)cetostearylether,
   • 4,82 % Sorbitan-monooleat,
   • 6 % Mannitol.

15. Emulsion nach einem der vorstehenden Ansprüche, **dadurch gekennzeichnet, dass** sie außerdem ein Alkylpolyglycosid umfasst.

**16.** Emulsion nach einem der vorstehenden Ansprüche, **dadurch gekennzeichnet, dass** sie außerdem ein Kälteschutzmittel umfasst.

**17.** Verwendung einer Emulsion nach einem der vorstehenden Ansprüche für die Herstellung einer immunogenen Zusammensetzung, die dazu vorgesehen ist, auf intramuskulärem Wege verabreicht zu werden.

**18.** Verwendung einer Emulsion nach einem der Ansprüche 1 bis 16 für die Herstellung einer immunogenen Zusammensetzung, die dazu vorgesehen ist, auf intradermalem Wege verabreicht zu werden.

**19.** Verwendung einer Emulsion nach einem der Ansprüche 1 bis 16 für die Herstellung einer immunogenen Zusammensetzung, die dazu vorgesehen ist, auf subkutanem Wege verabreicht zu werden.

**20.** Verwendung einer Emulsion nach einem der Ansprüche 1 bis 16 für die Herstellung einer immunogenen Zusammensetzung, die dazu vorgesehen ist, gegen Grippe verabreicht zu werden.

**21.** Verwendung einer Emulsion nach einem der Ansprüche 1 bis 16 für die Herstellung einer immunogenen Zusammensetzung, die dazu vorgesehen ist, gegen AIDS verabreicht zu werden.

**22.** Verwendung einer Emulsion nach einem der Ansprüche 1 bis 16 für die Herstellung einer immunogenen Zusammensetzung, die dazu vorgesehen ist, gegen Erkrankungen mit dem humanen Cytomegalovirus verabreicht zu werden.

**23.** Verfahren zur Herstellung einer immunogenen Zusammensetzung, die mindestens ein Impf-Antigen und eine Emulsion Öl-in-Wasser umfasst, **dadurch gekennzeichnet, dass** das Öl ein Squalen ist und die Öl-in-Wasser-Emulsion mittels eines Verfahrens der Phaseninversion durch Veranderung der Temperatur erhalten wird.

**24.** Verfahren nach dem vorstehenden Anspruch, **dadurch gekennzeichnet, dass** es mindestens eine Stufe zur Herstellung einer Emulsion Öl-in-Wasser durch Abkühlung einer inversen Emulsion Wasser-in-Öl umfasst, die ihrerseits umfasst:

- Squalen,
- ein wässeriges Lösungsmittel,
- ein hydrophiles nichtionisches oberflächenaktives Mittel, das ein Polyoxyethylen-alkylether ist, dessen Gleichgewicht hydrophil/lipophil (HLB) größer als oder gleich 10 ist,
- ein hydrophobes nichtionisches oberflächenaktives Mittel, dessen HLB niedriger als 9 ist.

**25.** Verfahren nach dem vorstehenden Anspruch, **dadurch gekennzeichnet, dass** man die inverse Emulsion Wasser-in-Öl erhält, indem man Squalen, ein wässeriges Lösungsmittel, ein nichtionisches oberflächenaktives Mittel, das ein Polyoxyethylen-alkylether ist, dessen Gleichgewicht hydrophil/lipophil (HLB) größer als oder gleich 10 ist, und ein hydrophobes nichtionisches oberflächenaktives Mittel, dessen HLB niedriger als 9 ist, miteinander vermischt, um zunächst eine grobe Emulsion Öl-in-Wasser zu erhalten, und man anschließend diese Emulsion bis auf mindestens die Inversions-Temperatur der Phase erwärmt, um die inverse Emulsion zu erhalten.

**26.** Verfahren nach Anspruch 24, gemäß dem man

- getrennt erwärmt auf eine Temperatur von mindestens gleich der Phasen-Inversions-Temperatur, einerseits eine wässerige Phase, umfassend ein wässeriges Lösungsmittel und ein oberflächenaktives Mittel, das ein Polyoxyethylen-alkylether ist, dessen Gleichgewicht hydrophil/lipophil (HLB) größer als oder gleich 10 ist, und andererseits eine ölige Phase, umfassend Squalen und ein hydrophobes nichtionisches oberflächenaktives Mittel, dessen HLB niedriger als 9 ist, und man anschließend
- die zwei Phasen vermischt, um eine inverse Emulsion Wasser-in-Öl zu erhalten.

**27.** Verfahren nach Anspruch 24, gemäß dem man

- getrennt erwärmt auf eine Temperatur von unterhalb der Phasen-Inversions-Temperatur der Emulsion, einerseits eine wässerige Phase, umfassend ein wässeriges Lösungsmittel und ein oberflächenaktives Mittel, das ein Polyoxyethylen-alkylether ist, dessen Gleichgewicht hydrophil/lipophil (HLB) größer als oder gleich 10 ist, und andererseits eine ölige Phase, umfassend Squalen und ein hydrophobes oberflächenaktives Mittel, dessen HLB niedriger als 9 ist, und man anschließend

- die zwei Phasen vermischt, um eine Emulsion Öl-in-Wasser zu erhalten, und man danach
- die erhaltene Emulsion Öl-in-Wasser auf eine Temperatur von mindestens gleich der Phasen-Inversions-Temperatur erwärmt, um eine inverse Emulsion Wasser-in-Öl zu erhalten.

28. Verfahren nach einem der Ansprüche 23 bis 27, **dadurch gekennzeichnet, dass** die Phasen-Inversions-Temperatur zwischen einschließlich 45 und 80 °C beträgt.

29. Verfahren nach dem vorstehenden Anspruch, **dadurch gekennzeichnet, dass** die Phasen-Inversions-Temperatur zwischen einschließlich 50 und 65 °C beträgt.

30. Verfahren nach einem der Ansprüche 23 bis 29, **dadurch gekennzeichnet, dass** es außerdem mindestens eine Stufe der Lyophilisation umfasst.

31. Immunogene Zusammensetzung, **dadurch gekennzeichnet, dass** sie durch das Verfahren nach einem der Ansprüche 23 bis 30 erhalten werden kann.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6299884 B
- WO 9933346 A
- US P5834307 A
- US 6100064 A
- WO 9605294 A

**Littérature non-brevet citée dans la description**

- **RASMUSSEN L et al.** *J. Virol.,* 1985, vol. 55, 274-280
- **C.W. POTTER.** *Vaccine,* 2003, vol. 21, 940-5